# EUROPEAN PATENT SPECIFICATION

(11) **EP 1 824 830 B1**
(45) Date of publication and mention of the grant of the patent: **14.07.2010**
(21) Application number: 05807786.8
(22) Date of filing: 17.11.2005
(51) Int. Cl.: C07D 215/40, A61K 31/47, A61P 25/00, C07B 59/00, A61K 51/04

(54) **RADIOLABELLED QUINOLINE-BASED LIGANDS FOR THE 5-HT6 RECEPTOR FUNCTIONALITY**
RADIOAKTIV MARKIERTE LIGANDEN AUF CHINOLINBASIS FÜR DIE 5-HT6-REZEPTORFUNKTIONALITÄT
LIGANDS RADIOMARQUÉS À BASE DE QUINOLÉINE POUR LA FONCTIONNALITÉ DU RÉCEPTEUR 5-HT6

(30) Priority: 19.11.2004 GB 0425548
(43) Date of publication of application: 29.08.2007
(73) Proprietor: GLAXO GROUP LIMITED, Greenford, Middlesex UB6 0NN (GB)
(72) Inventor: GEE, Antony David, GlaxoSmithKline, Cambridge, Cambridgeshire CB2 2GG (GB); MARTARELLO, Laurent, c/o GlaxoSmithKline, Cambridge, Cambridgeshire CB2 2GG (GB); JOHNSON, Christopher Norbert, GlaxoSmithKline, Harlow, Essex CM19 5AW (GB); WITTY, David R., GlaxoSmithKline, Harlow, Essex CM19 5AW (GB)
(74) Representative: Griffith, Johanna Elise
(86) International application number: PCT/EP2005/012463
(87) International publication number: WO 2006/053785

(56) References cited:
- WO-A-03/080580
- WO-A-03/080608
- WO-A-2005/021530
- WO-A-2005/026125
- WO-A-2005/030724
- WO-A-2005/113539
- HIRST ET AL: "Characterization of [125]-SB-258585 binding to human recombinant and native 5-HT6 receptors in rat, pig and human brain tissue" BRITISH JOURNAL OF PHARMACOLOGY, vol. 130, 2000, pages 1597-1605, XP002362259 cited in the application
- EAST ET AL: "5HT6 receptor binding sites in schizophrenia and following antipsychotic drug administration: Autoradiographic studies with [125I]SB-258585" SYNAPSE, vol. 45, 2002, pages 191-199, XP002362260
- ROBERTS ET AL: "The distribution of 5-HT6 receptors in rat brain: an autoradiographic binding study using the radiolabelled 5-HT6 receptor antagonist [125I]SB-258585" BRAIN RESEARCH, vol. 934, 2002, pages 49-57, XP002362261
- MARTARELLO ET AL: "Radiolabelling and in vivo evaluation of [11C]GSK215083 as potential PET radioligand for the 5-HT6 receptor in the porcine brain" JOURNAL OF CEREBRAL BLOOD FLOW & METABOLISM, vol. 25, 2005, page S598, XP002362167
- MARTARELLO ET AL: "Radiolabelling and in vivo evaluation of [11C]GSK215083 as a potential PET radioligand for the 5-HT6 receptor in the porcine brain" J. LABEL. COMPD.RADIOPHARM., vol. 48, 2005, page S7, XP002362168

## Description

The present invention relates to a radiolabelled ligand for 5-hydroxytryptamine-6 (5-HT₆) receptors, useful for the labelling and diagnostic imaging of 5-HT₆ receptor functionality.

Noninvasive, nuclear imaging techniques can be used to obtain basic and diagnostic information about the physiology and biochemistry of living subjects, including experimental animals, patients and volunteers. These techniques rely on the use of imaging instruments that can detect radiation emitted from radiotracers administered to living subjects. The information obtained can be reconstructed to provide planar and tomographic images which reveal the distribution and/or concentration of the radiotracer as a function of time.

Positron emission tomography (PET) is a noninvasive imaging technique that offers the highest spatial and temporal resolution of all nuclear medicine imaging modalities and has the added advantage that it can allow for true quantitation of tracer concentrations in tissues. The technique involves the use of radiotracers, labelled with positron emitting radionuclides, that are designed to have *in vivo* properties which permit measurement of parameters regarding the physiology or biochemistry of a variety of processes in living tissue.

Compounds can be labelled with positron or gamma emitting radionuclides. The most commonly used positron emitting radionuclides are ¹⁵O, ¹³N, ¹¹C and ¹⁸F, which are accelerator produced and have half lifes of 2, 10, 20 and 110 minutes respectively. The most widely used gamma emmitting radionuclides are 18^{F}, ^{99m}Tc, ²⁰¹TI and ¹²³I_{.}

*In vitro* studies using 5-HT₆ receptor antagonists selectively binding with high affinity to recombinant and native 5-HT₆ receptors have localised 5-HT₆ receptors almost exclusively in the CNS (W. D. Hirst et al., Br. J. Pharmacol., 130, 1597-1605 (2000), A. J. Sleight et al., Br. J. Pharmacol., 124, 556-562 (1998)). As a therapeutic target the 5-HT₆ receptor has been investigated for a variety of CNS disorders including anxiety, epilepsy, cognitive function, dementia psychosis and affective disorders.

WO 03/080580 discloses a series of quinoline compounds said to be 5-HT₆ receptor antagonists and claimed to be useful in the treatment of various CNS disorders. When Example 7 (GSK215083) of WO 03/080580 is radiolabelled it has been found to allow *in vivo* imaging of 5HT-6 receptors in the brain.

Accordingly the present invention provides [¹¹C-*N*-methyl]3-[(3-fluorophenyl)sulfonyl]-8-(4-methyl-1-piperazinyl)quinoline.

The present invention also provides a radiopharmaceutical composition which comprises [¹¹C-*N*-methyl]3-[(3-fluorophenyl)sulfonyl]-8-(4-methyl-1-piperazinyl)quinoline. and a pharmaceutically acceptable carrier or excipient.

Preferably, in the methods of the present invention the mammal is human.

The present invention also relates to a process for the preparation of [¹¹C-*N*-methyl] 3-[(3-fluorophenyl)sulfonyl]-8-(4-methyl-1-piperazinyl)quinoline which comprises reacting 3-[(3-fluorophenyl)sulfonyl]-8-(1-piperazinyl)quinoline with [¹¹C]methyl triflate.

Compound (I) may be used in clinical studies to evaluate the role of 5-HT₆ receptor ligands in a variety of disease areas where 5-HT₆ receptor ligands are believed to be involved.

Scheme 1 represents a synthetic route towards compounds of formula (I) wherein R_{1'} is a leaving group and R_{2'} is F or R_{1'} is methyl and R_{2'} is a leaving group.

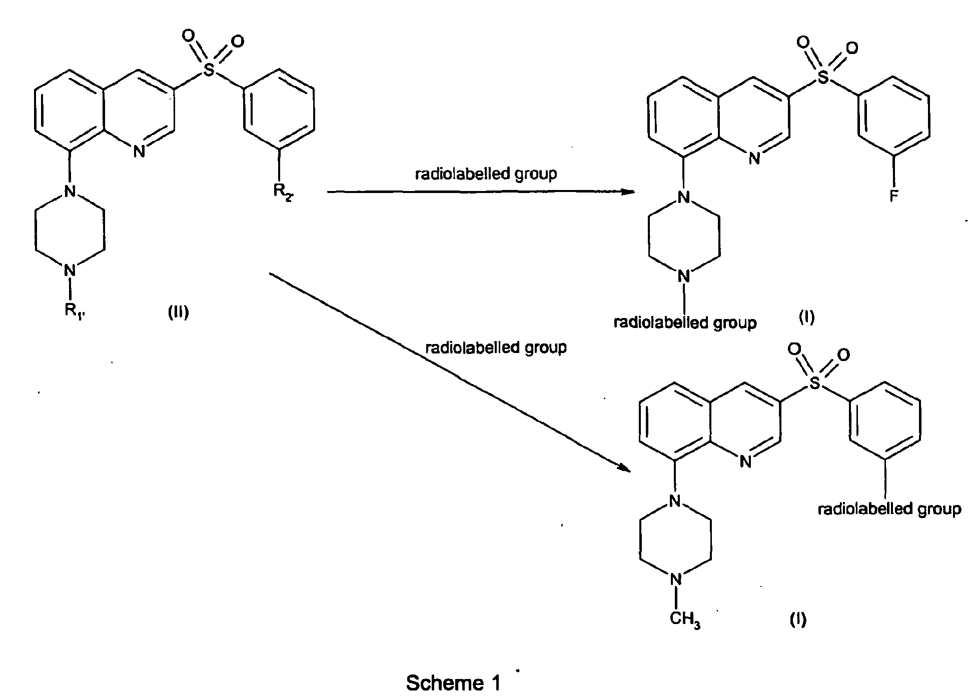

One synthetic route for the synthesis of a compound of formula (II) is shown in Scheme 2.

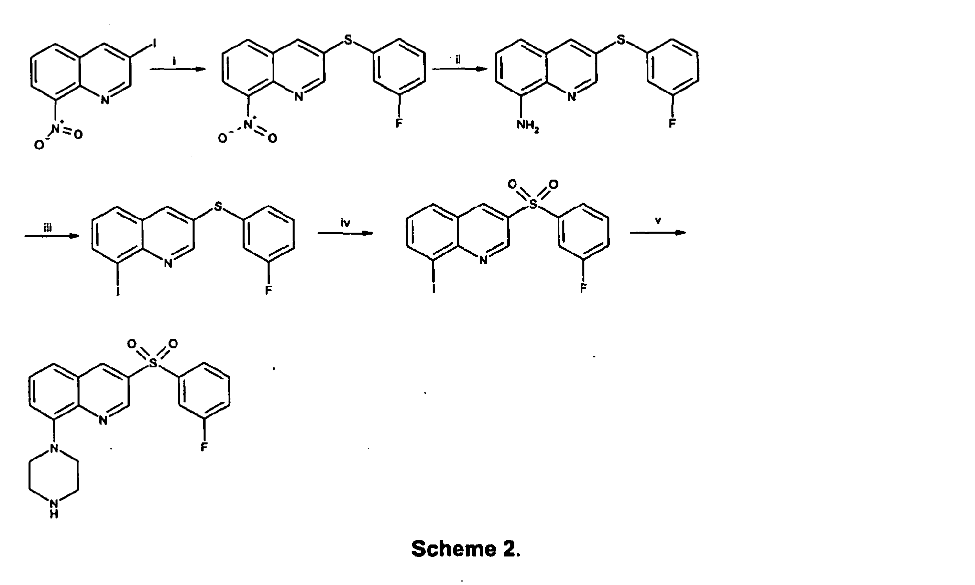

Steps invoved in the preparation of compound (II) are i: Copper(I) iodide, potassium phosphate, ethylene glycol, 3-fluorobenzenethiol, 80°C, 3.5 h; ii: iron powder, acetic acid, 60 °C, 5 h; iii: trifluoroacetic, *n*-butylnitrite then tetra-*n*-butylammonium iodide, r.t, 1 h; iv: monomagnesium peroxyphthalate hexahydrate, 40°C, 12 h; and v: Pd₂(dba)₃ , 1,1'-bis-diphenylphosphenoferrocene, sodium *tert*butoxide, piperazine, 40°C, 16½h.

The starting materials and other reagents are available commercially or can be synthesised by well-known and conventional methods.

### Example 1

### [¹¹C-N-methyl]-3-(3-Fluoro-benzenesulfonyl)-8-(4-methyl-piperazin-1-yl)-quinoline ([¹¹C]GSK215083)

The production of [¹¹C]CO₂ via the ¹⁴N(p,α)¹¹C reaction was carried out by irradiation of a nitrogen target (N₂, 99.99%) with 0.5% O₂ (99.99%) at a 17 MeV cyclotron (General Electric PET-trace). [¹¹C]CH₃I was prepared by catalytic reduction (Ni) of [¹¹C]CO₂ to [¹¹C]CH₄ followed by gas phase iodination with I₂ using the PETtrace Mel MicroLab system(General Electric). Subsequently [¹¹C]CH₃I was passed through a quartz tube loaded with silver triflate heated at 195°C for conversion to [¹¹C]MeOTf.

The [¹¹C]methyl triflate was delivered to reaction containing compound (II) and 2,2,6,6-tetramethylpiperidine in methanol:acetonitrile at room temperature. Following that first step, the reaction mixture was heated at 85°C for 5 min and then injected onto a semi-preparative column for purification. For all radiosyntheses the radiochemical purity of the product was >99%. The radiochemical yield (decay-corrected, related to [¹¹C]methyliodide) ranged between 50 and 60% and the specific activity was >200 GBq/µmol. The average time for synthesis was 40 min from the end of beam (EOB) including HPLC purification and formulation.

The title compound was obtained by methylation of the corresponding desmethyl precursor compound (II) with [¹¹C]MeoTf. The [¹¹C]MeOTf produced was trapped at room temperature into a 1 ml glass container loaded with compound (II) (1 mg) 2,2,6,6-tetramethylpiperidine (10 µl) in MeOH:acetonitrile, 2:1 (300 µl). After trapping the reaction mixture mixture was heated 5 min at 80 °C and injected onto a semi-preparative column for purification. The title compound was purified on a C18 column (Sphereclone ODS(2) C-18 250 x 7.4 mm); using ACN:70 mM NaH2PO4 (60:40) as mobile phase at a flow rate of 9 ml/min, the title compound eluted at 10.5 min with baseline separation between the product and the starting material. The product fraction collected after 10min was evaporated to dryness, and reformulated in 10 ml of 0.9% NaCl. Quality controls were performed on a Sphereclone ODS(2) C-18 250 x 4.6 mm using ACN:70 mM NaH2PO4 (60:40) as mobile phase at a flow rate of 2 ml/min, the title compound eluted at 3.5 min.

### Biological Data

### 1. In vitro activity

GSK215083 has very high affinity for the human recombinant 5-HT₆ receptor stably expressed in the HeLa cell line, with a pKᵢ value of 9.82 (competition studies with [³H]LSD as a radioligand) and is selective for the human 5-HT₆ receptor over other receptors screened to date.

**Table 1. Binding Profile (pKi values) of GSK215083**

| **Receptor** | **pKᵢ** | **Receptor** | **pKᵢ** |
|---|---|---|---|
| 5-HT_{1A} | <5.15 | 5-HT₆ | 9.82 |
| 5-HT_{1B} | 6.98 | 5-HT₇ | <5 |
| 5-HT_{1D} | 7.16 | α_{1B} adrenoreceptor | 6.01 |
| 5-HT_{2A} | 9.14 | Dopamine D₂ | 6.04 |
| 5-HT_{2B} | 8.04 | Dopamine D₃ | 6.01 |
| 5-HT_{2C} | 7.75 | | |

### 2. 5-HT₆ localisation in the brain

Yorkshire/Danish Landrace porcines (- 40 Kg; n=4) were housed singly in thermostatically controlled (20 °C) and naturally illuminated stalls. The pigs were provided with Soavl® chow (DLG, Denmark) and water *ad libitum* by trough. Animals were fasted for 24 hours prior to PET examination, with free access to water during this interval. Animals were anaesthetised by induction with ketamine and midazolam (both intramuscular and intravenous (i.v.)) and maintained in deep anaesthesia using isoflurane (1 - 2 %; Abbott, Denmark). The left femoral artery and vein of each animal were surgically cannulated using catheters (Avanti® size 4F-7F). The femoral artery for blood sampling and blood pressure (BP) recordings and the femoral vein for administration of radiolabelled and non-labelled agents. Animals were placed supine in a Siemens ECAT EXACT HR tomograph, with the head immobilised in a custom-made holding device. During the study, blood pH, *p*CO₂ and *p*O₂ levels were monitored and maintained within the normal physiological range. In addition, BP and heart rate were recorded throughout the study. At the end of each study day, the animal was terminated via an intravenous dose of sodium pentobarbital (20 ml). [¹¹C]GSK215083 was administered intravenously in the femoral vein as a 1 minute bolus injection. PET scanning and arterial blood sampling was commenced upon start of the radioligand administration.

[¹¹C]GSK215083 readily enters the brain reaching peak regional tissue concentrations at approximately 20min post injection followed by a slow washout from brain regions known to be rich in 5-HT₆ receptors with highest uptake and retention observed in striatum. The observed rank order of regional brain concentrations was striatum>cortical regions>cerebellum, consistent with reported 5-HT₆ receptor densities and localisation determined by tissue section autoradiograpgy in animals and man. [¹¹C]GSK215083 concentation was low in cerebellum, a brain region known to possess very low level of 5-HT₆ receptors.

### 3. Saturation of 5-HT₆ receptors in the brain

Four sequential high specific activity iv radioligand ([¹¹C]GSK215083) administrations were performed in same animal, same day. Following a baseline scan, [¹¹C] GSK215083 was coadministered with escalating dose of unlabelled GSK215083 (0.005, 0.05 and 0.5mg/kg). [¹⁵O]CO and [¹⁵O]H2O were administered pre and post administration of GSK215083 to monitor and to correct for changes in cerebral blood volume and changes in cerebral blood flow. Upon injection of [¹¹C]GSK215083, striatum to cerebellum and cortex to cerebellum ratios of 2 to 1 and 1.5 to 1, respectively were reached at 60 min post injection. In contrast, the same ratios decreased to 1.5 to 1 and 1.2 to 1 at 60 min post coinjection with 0.005mg/kg of GSK215083. When 0.05mg/kg of GSK215083 was administered striatum to cerebellum and cortex to cerebellum ratios further decreased to 1.3 to 1 and 1.1 to 1, respectively. Finally, ratios of 1.1 to 1 and 1 to 1, respectively were measured 60min post administration of 0.5mg/kg. The dose dependent decrease in [¹¹C]GSK215083 signal in brain regions known to be rich in 5-HT₆.

## Claims

1. [¹¹C-N-methyl]-3-(3-Fluoro-benzenesulfonyl)-8-(4-methyl-piperazin-1-yl)-quinoline

2. A pharmaceutical composition which comprises a compound according to claim 1.

3. A process for the preparation of the compound according to claim 1, which comprises reacting 3-[(3-fluorophenyl)sulfonyl]-8-(1-piperazinyl)quinoline with [¹¹C]methyl triflate.

4. A method for detecting the binding of a test compound to 5-HT₆ receptors comprising the use of compounds of compound (I) according to claim 3.

## Patentansprüche

1. [¹¹C-N-Methyl]-3-(3-fluorbenzolsulfonyl)-8-(4-methylpiperazin-1-yl)chinolin.

2. Ein Arzneimittel, welches eine Verbindung gemäß Anspruch 1 umfasst.

3. Ein Verfahren zur Herstellung einer Verbindung gemäß Anspruch 1, welches das Umsetzen von 3-[(3-Fluorphenyl)sulfonyl]-8-(1-piperazinyl)chinolin mit [¹¹C]-Methyltriflat umfasst.

4. Ein Verfahren zur Ermittlung der Bindung einer Testverbindung an 5-HT₆-Rezeptoren, umfassend die Verwendung von Verbindungen der Verbindung (I) gemäß Anspruch 3.

## Revendications

1. [¹¹C-N-méthyl]-3-(3-fluoro-benzènesulfonyl)-8-(4-méthyl-pipérazine-1-yl)-quinoléine.

2. Composition pharmaceutique qui comprend un composé suivant la revendication 1.

3. Procédé pour la préparation du composé suivant la revendication 1, qui comprend la réaction de 3-[(3-fluoro-phényl)sulfonyl]-8-(1-pipérazinyl) quinoléine avec du triflate de [11C] méthyle .

4. Méthode pour la détection de la liaison d'un composé d'essai aux récepteurs 5-HT₆, comprenant l'utilisation de composés du composé (I) suivant la revendication 3.
